# EUROPEAN PATENT SPECIFICATION

(11) **EP 2 558 857 B1**
(45) Date of publication and mention of the grant of the patent: **06.09.2017**
(21) Application number: 11735533.9
(22) Date of filing: 15.04.2011
(51) Int. Cl.: G01N 33/50, C12N 5/0783

(54) **NEW METHODS FOR ISOLATING TR1 CELLS**
NEUES VERFAHREN ZUR ISOLIERUNG VON TR1-ZELLEN
NOUVEAUX PROCÉDÉS POUR ISOLER DES CELLULES TR1

(30) Priority: 15.04.2010 US 324450 P; 15.04.2010 EP 10368021
(43) Date of publication of application: 20.02.2013
(73) Proprietor: TXCell, 06560 Valbonne (FR)
(72) Inventor: FOUSSAT, Arnaud, F-06140 Biot (FR); BRUN, Valérie, F-06140 Biot (FR); BELMONTE, Nathalie, F-06200 Nice (FR); BASTIAN, Hervé, F-06810 Auribeau-sur Siagne (FR); QUATTANNENS, Brigitte, F-06210 Mandelieu-la Napoule (FR)
(74) Representative: Icosa
(86) International application number: PCT/IB2011/001232
(87) International publication number: WO 2011/128779

(56) References cited:
- WO-A1-2009/036521
- BERNARD F ET AL: "Lymphocytes T regulateurs et maladies auto-immunes systemiques : lupus erythemateux systemique, polyarthrite rhumatoide et syndrome de Gougerot-Sjogren primaire", REVUE DE MEDECINE INTERNE, CMR, ASNIERES, FR LNKD- DOI:10.1016/J.REVMED.2009.03.364, vol. 31, no. 2, 1 February 2010 (2010-02-01), pages 116-127, XP026880453, ISSN: 0248-8663 [retrieved on 2009-12-03]
- BRUSKO TODD M ET AL: "Human regulatory T cells: role in autoimmune disease and therapeutic opportunities.", IMMUNOLOGICAL REVIEWS JUN 2008 LNKD- PUBMED:18613848, vol. 223, June 2008 (2008-06), pages 371-390, XP002596194, ISSN: 1600-065X
- RUTELLA SERGIO ET AL: "Regulatory T cells and tolerogenic dendritic cells: from basic biology to clinical applications.", IMMUNOLOGY LETTERS 15 JUN 2004 LNKD- PUBMED:15234530, vol. 94, no. 1-2, 15 June 2004 (2004-06-15), pages 11-26, XP002596191, ISSN: 0165-2478
- VENKEN K ET AL: "Disturbed regulatory T cell homeostasis in multiple sclerosis", TRENDS IN MOLECULAR MEDICINE, ELSEVIER CURRENT TRENDS, GB LNKD- DOI:10.1016/J.MOLMED.2009.12.003, vol. 16, no. 2, 1 February 2010 (2010-02-01), pages 58-68, XP026904283, ISSN: 1471-4914 [retrieved on 2010-02-12]
- CHEN MEI-LING ET AL: "Regulatory T cells suppress tumor-specific CD8 T cell cytotoxicity through TGF-beta signals in vivo.", PROCEEDINGS OF THE NATIONAL ACADEMY OF SCIENCES OF THE UNITED STATES OF AMERICA 11 JAN 2005 LNKD- PUBMED:15623559, vol. 102, no. 2, 11 January 2005 (2005-01-11), pages 419-424, XP002596192, ISSN: 0027-8424
- CRISTINA MARIA COSTANTINO ET AL: "Multiple Sclerosis and Regulatory T Cells", JOURNAL OF CLINICAL IMMUNOLOGY, KLUWER ACADEMIC PUBLISHERS-PLENUM PUBLISHERS, NE LNKD- DOI:10.1007/S10875-008-9236-X, vol. 28, no. 6, 2 September 2008 (2008-09-02), pages 697-706, XP019644933, ISSN: 1573-2592
- TRZONKOWSKI PIOTR ET AL: "Ex vivo expansion of CD4(+)CD25(+) T regulatory cells for immunosuppressive therapy.", CYTOMETRY. PART A : THE JOURNAL OF THE INTERNATIONAL SOCIETY FOR ANALYTICAL CYTOLOGY MAR 2009 LNKD- PUBMED:18855921, vol. 75, no. 3, March 2009 (2009-03), pages 175-188, XP002596193, ISSN: 1552-4930

## Description

### FIELD OF THE INVENTION

The present invention is related to methods for identifying and/or isolating Tr1 cells, resting Tr1 cells and/or activated Tr1 cells, to enriched populations of Tr1 cells, resting Tr1 cells and/or activated Tr1 cells, to depleted populations in Tr1 cells, resting Tr1 cells and/or activated Tr1 cells and to methods and kits for diagnosing or treating chronic inflammatory diseases, autoimmune diseases, allergic diseases, cancer and organ transplantation conditions.

### BACKGROUND OF THE INVENTION

Homeostasis of the immune system depends on the balance between immune response to an invading pathogen and immune tolerance to self antigens. Both central and peripheral tolerances are important mechanisms for the induction and maintenance of T cell tolerance. In the last decade, much attention has been paid to regulatory T cells (Treg) that play a significant role in maintaining peripheral immune tolerance. It has now been firmly established that Treg can be divided into two different subtypes: natural Treg (nTreg) and inducible Treg populations such as TGF-beta induced Treg cells, Tr1 cells or Th3 cells. As these Treg populations play a major role in peripheral immune tolerance, it has become a crucial issue to be able to identify, isolate, or enrich these different Treg subsets in order to facilitate diagnosis, assessment or treatment of immunological conditions such as chronic inflammatory diseases, autoimmune diseases or allergic diseases.

For example, since their identification, nTreg have been identified by the expression of CD25. However, CD25 is an activation marker of conventional T cells (convT) and thus is not exclusive to Treg cells. Recent advances in the discrimination of human nTreg have been made by identification of cell surface or intracellular markers such as Foxp3, CD127 (WO2007140472), CD45RA (WO2007/117602), CD134 (WO2009/036521) and CD49d (WO2009/047003). Regarding Tr1 cells, their specific identification is still difficult and remains based mainly on IL-10 secretion determination. There is thus a need to provide means for a better and more accurate discrimination of Tr1 cells.

In addition, Tr1 cells have proven to be useful in cell therapy, as injection of Tr1 cells in patients having Crohn's disease ameliorated their condition. There is thus a need for identifying and isolating more accurately Tr1 cells in view of their use in cell therapy.

The inventors have found that Tr1 cells may be specifically identified using the CD127 and CD62L cell surface markers. Indeed, the use of these two markers in addition to the CD4 marker and optionally the CD25 marker allows the discrimination of Tr1 cells among conventional resting and activated T cells and resting and activated nTreg.

### SUMMARY OF THE INVENTION

One object of the invention is a method of identifying a Tr1 cell population, comprising detecting the cell surface expression of CD4, CD127 and CD62L markers on a cell population, wherein the cells expressing:
- CD4 and
- a low level of CD127 and
- a low level CD62L,
are the Tr1 cells.

Another object of the invention is a method of identifying a resting Tr1 cell population, comprising detecting the cell surface expression of CD4, CD25, CD127 and CD62L markers on a cell population, wherein the cells expressing:
- CD4 and
- a low level of CD127,
- a low level of CD62L,
and not expressing:
- CD25,
are the resting Tr1 cells.

Another object of the invention is a method of identifying an activated Tr1 cell population, comprising detecting the cell surface expression of CD4, CD25, CD127 and CD62L markers on a cell population, wherein the cells expressing:
- CD4,
- CD25,
- a low level of CD127 and
- a low level of CD62L,
are the activated Tr1 cells.
Another object of the invention is a method for isolating a resting or an activated Tr1 cell population, comprising identifying a resting Tr1 cell population or an activated Tr1 cell population as described here above, and isolating said population.
Another object of the invention is an isolated population of resting Tr1 cells or of activated Tr1 cells obtained by the method as described here above.

Another object of the invention is an isolated population of resting Tr1 cells having the phenotype CD4⁺CD25⁻CD127^{low}CD62L^{low}.
Another object of the invention is an isolated population of activated Tr1 cells having the phenotype CD4⁺CD25+CD127^{low}CD62L^{low}.
Another object of the invention is a method for enriching a cell population in Tr1 cells, comprising:
- identifying the cells expressing CD4, a low level of CD127 and a low level of CD62L,
- selecting said cells,
thereby obtaining a cell population enriched in Tr1 cells wherein the percentage of Tr1 cells is at least twice the percentage of Tr1 cells before enrichment.
Another object of the invention is a method for enriching a cell population in resting Tr1 cells, comprising:
- identifying the cells expressing CD4 and a low level of at least one of CD127 and CD62L, and not expressing CD25,
- selecting said cells,
thereby obtaining a cell population enriched in resting Tr1 cells wherein the percentage of resting Tr1 cells is at least twice the percentage of resting Tr1 cells before enrichment.
Another object of the invention is a method for enriching a cell population in activated Tr1 cells, comprising:
- identifying the cells expressing CD4, CD25 and expressing a low level of CD127 and a low level of CD62L,
- selecting said cells,
thereby obtaining a cell population enriched in activated Tr1 cells wherein the percentage of activated Tr1 cells is at least twice the percentage of activated Tr1 cells before enrichment.
Another object of the invention is a method for enriching a cell population in resting and/or activated Tr1 cells, comprising identifying the resting and/or activated Tr1 cells as described here above, and selecting said cells, thereby obtaining a cell population enriched in resting and/or activated Tr1 cells wherein the percentage of resting and/or activated Tr1 cells is at least twice the percentage of resting and/or activated Tr1 cells before enrichment.

Another object of the invention is an enriched population of resting Tr1 having the phenotype CD4⁺CD25⁻CD127^{low}CD62L^{low} or activated Tr1 cells having the phenotype CD4⁺CD25⁺CD127^{low}CD62L^{low}, wherein at least 50% of the cells of the enriched population of resting or activated Tr1 cells are Tr1 cells.
Another object of the invention is an enriched population of resting Tr1 having the phenotype CD4⁺CD25⁻CD127^{low}CD62L^{low} and activated Tr1 cells having the phenotype CD4⁺CD25⁺CD127^{low}CD62L^{low}, wherein at least 50% of the cells of the enriched population of resting and activated Tr1 cells are Tr1 cells.
Another object of the invention is a method for depleting a cell population in resting Tr1 cells and/or activated Tr1 cells, comprising identifying the resting Tr1 cells and/or the activated Tr1 cells as defined here above, and depleting said cells, thereby obtaining a cell population depleted in resting and/or activated Tr1 cells, wherein the percentage of resting and/or activated Tr1 is at most 0.75-fold the percentage of resting and/or activated Tr1 cells before depletion.
Another object of the invention is a pharmaceutical composition comprising an isolated resting and/or activated Tr1 cell population as described here above or an enriched resting and/or activated Tr1 cell population as described here above.
In one embodiment, the pharmaceutical composition comprising an isolated resting and/or activated Tr1 cell population as described here above or an enriched resting and/or activated Tr1 cell population as described here above is for preventing or treating an immune response associated with an infection or transplantation or an allergic disease or for preventing or treating an inflammatory condition or an autoimmune condition.
Another object of the invention is a pharmaceutical composition comprising a mixture of resting Tr1 cells and activated Tr1 cells, wherein the resting Tr1 cells have the phenotype CD4⁺CD25⁻CD127^{low}CD62L^{low} and the activated Tr1 cells have the phenotype CD4⁺CD25⁺CD127^{low}CD62L^{low}.
In one embodiment, said pharmaceutical composition is for treating an immune response associated with an infection or transplantation or an allergic disease or for preventing or treating an inflammatory condition or an autoimmune condition. Another object of the invention is a method for monitoring in vitro the effect of a therapy in a subject, comprising identifying the resting and/or activated Tr1 cell population in a biological sample obtained from the subject before and after therapy according to the method as described here above, wherein an increase in the resting and/or activated Tr1 cell population after therapy corresponds to a response to therapy.

### DETAILED DESCRIPTION OF THE INVENTION

Tr1 cells are a distinct cell population that can be characterized by a specific cytokine secretion profile as described here after. Tr1 cells are differentiated from naive CD4⁺ T cells that have encountered the antigen. Tr1 cells may thus be found in vivo under two states: activated and resting. As mentioned here above, discriminating Tr1 cells and in particular resting and activated Tr1 cells by using surface markers is currently not possible as a specific surface phenotype for these populations is not available.

The inventors surprisingly found that resting Tr1 cells present the following phenotype CD4⁺CD25⁻CD127^{lo/-}CD62L^{lo/-} and activated Tr1 cells present the following phenotype CD4⁺CD25⁺CD127^{lo/-}CD62L^{lo/-}. This was particularly surprising as it is well known in the art that resting T cells typically express high levels of CD62L.

The invention provides methods for identifying, isolating, enriching and/or depleting human Tr1 cells, and in particular human resting Tr1 cells and human activated Tr1 cells.
The invention also provides the resultant Tr1 cells, resting Tr1 cells and activated Tr1 cells populations or compositions and methods of use.

One object of the invention is a method of identifying a Tr1 cell population, comprising detecting the cell surface expression of CD4, CD127 and CD62L markers on a cell population, wherein the cells that express CD4, a low level of CD127 and a low level of CD62L, are the Tr1 cells.
Another object of the invention is a method of identifying a resting Tr1 cell population, comprising detecting the cell surface expression of CD4, CD25, and at least one of CD127 and CD62L markers on a cell population, wherein the cells that express CD4, a low level of at least one of CD127 and CD62L, and do not express CD25, are the resting Tr1 cells.
Another object of the invention is a method of identifying an activated Tr1 cell population, comprising detecting the cell surface expression of CD4, CD25, CD127 and CD62L markers on a cell population, wherein the cells that express CD4, CD25, a low level of CD127 and a low level of CD62L are the activated Tr1 cells.
Another object of the invention is a method of identifying a resting Tr1 cell population and/or an activated Tr1 cell population among conventional resting and activated T cells and natural regulatory resting and activated T cells, wherein the resting Tr1 cells are CD4⁺CD25⁻CD127^{lo/-}CD62L^{lo/-} and the activated Tr1 cells are CD4⁺CD25⁺CD127^{lo/-}CD62L^{lo/-}.

The inventors hereby demonstrate that Tr1 cells can be specifically identified using the CD4, CD127 and CD62L markers. More specifically, the inventors also show that resting Tr1 cells can be discriminated from activated Tr1 cells, using the CD4, CD25, CD127 and CD62L markers. As used herein, the term "marker" refers to proteins, carbohydrates, or lipids on the surface of the cells that can be used to discriminate a cell population.

As used herein, the term "expression" refers interchangeably to expression of a gene or gene product, including the encoded polypeptide or protein. Expression of a gene product may be determined, for example, by immunoassay using one or more antibody(ies) that bind with the polypeptide. Alternatively, expression of a gene may be determined by measurement of mRNA levels, for example, by RT-PCR, qPCR.
The term "naive" is well known in the art and refers to an immune cell or a population of cells that have not yet encountered any specific antigen. Naive T cells are resting cells.
The term "resting" is well known in the art and refers to an immune cell or a population of cells that do not proliferate, do not produce cytokines and that do not express conventional immune cell activation molecules at the surface such as CD25. Resting T cells may be naive T cells or memory T cells.
The term "activated" is well known in the art and refers to an immune cell or a population of cells which proliferates and/or produces cytokines and expresses conventional immune cell activation molecules at its surface such as CD25. Especially for T lymphocytes, the passage from a resting to an activated status is mediated by the encounter of the T cell with its specific antigen or by activation with activating cytokines or mitogens.
The term "low" or "lo" or "lo/-" as used in relation to CD127^{lo/-}, CD62L^{lo/-} or CD25L^{lo/-} is well known in the art and refers to the expression level of the cell marker of interest, in that the expression level of the cell marker is low by comparison with the expression level of that cell marker in the population of cells being analyzed as a whole. More particularly, the term "lo" refers to a distinct population of cells that express the cell marker at a lower level than one or more other distinct population of cells.
The term "high" or "hi" or "bright" is well known in the art and refers to the expression level of the cell marker of interest, in that the expression level of the cell marker is high by comparison with the expression level of that cell marker in the population of cells being analyzed as a whole.
The terms "+" and "-" are well known in the art and refer to the expression level of the cell marker of interest, in that the expression level of the cell marker corresponding to "+" is high or intermediate, also referred as "+/-", and the expression level of the cell marker corresponding to "-" is low or null. Generally, cells in the top 2, 3, 4, or 5% of staining intensity are designated "hi", with those falling in the top half of the population categorized as being "+". Those cells falling below 50%, of fluorescence intensity are designated as "lo" cells and below 5% as "-" cells.
As used herein, the term "CD127" refers to the "interleukin-7 receptor," present on a Tr1 cell surface. The IL-7 receptor alpha chain is described in the literature (e.g., Goodwin et al. (1990) Cell 60:941-951). IL-7R is also referred to in the literature as CD127. "CD127⁺" refers to cells which stain intermediate or brightly when treated with a labeled antibody directed toward CD127. "CD127^{lo/-}" refers to cells of a type that stains slightly/dully or not at all when contacted with a labeled CD127 antibody. Generally, the cells are distinguished according to their CD127 expression levels based upon a readily discernible difference in staining intensity as it is known to one of ordinary skill in the art. In some embodiments, the cut off for designating a cell as a CD127^{lo/-} cell can be set in terms of the fluorescent intensity distribution observed for all the cells with those cells falling below the 50%, 40%, 30% or 20% of fluorescence intensity being designated as CD127^{lo/-} cells. A CD127⁻ cell can be designated as one that falls below the tenth bottom percentile with respect to fluorescence intensity. In some embodiments, the frequency distribution of the CD127 staining is obtained for all the cells and the population curve fit to a higher staining and lower staining population, and cells assigned to the population to which they most statistically are likely to belong in view of a statistical analysis of the respective population distributions. In some embodiments, the CD127^{lo/-} cells stain two to three-fold less intensely than the CD127⁺ cells.
As used herein, the term "CD62L" refers to L-selectin which is a critical adhesion molecule for lymphocyte migration. "CD62L⁺" refers to cells which stain intermediate or brightly when treated with a labeled antibody directed toward CD62L. "CD62L^{lo/-"} refers to cells of a type which stains slightly/dully or not at all when contacted with a labeled CD62L antibody. Generally, the cells are distinguished according to their CD62L expression levels based upon a readily discernible difference in staining intensity as it is known to one of ordinary skill in the art. In some embodiments, the cut off for designating a cell as a CD62L^{lo/-} cell can be set in terms of the fluorescent intensity distribution observed for all the cells with those cells falling below the 50%, 40%, 30% or 20% of fluorescence intensity being designated as CD62L^{lo/-} cells. A CD62L⁻ cell can be designated as one which falls below the tenth bottom percentile with respect to fluorescence intensity. In some embodiments, the frequency distribution of the CD62L staining is obtained for all the cells and the population curve fit to a higher staining and lower staining population, and cells assigned to the population to which they most statistically are likely to belong in view of a statistical analysis of the respective population distributions. In some embodiments, the CD62L^{lo/-} cells stain two to three-fold less intensely than the CD62L⁺ cells.
As used herein, the term "CD4" refers to a cell-surface glycoprotein typically found on the mature helper T cells and immature thymocytes, as well as on monocytes and macrophages. On T cells, CD4 is the co-receptor for the T cell receptor (TCR) and recruits the tyrosine kinase lck. With its D1-portion, CD4 can attach to the beta2-domain of MHC class II molecules. "CD4⁺" refers to cells which stain brightly when contacted with labeled anti-CD4 antibody, and "CD4⁻" refers to cells of a type which stain the least brightly, dull or not at all, when contacted with a fluorescently labeled CD4 antibody. Generally, the cells are distinguished according to their CD4 expression levels based upon a readily discernible difference in staining intensity as the CD4 staining is clearly bimodal. In some embodiments, the frequency distribution of the CD4 staining is obtained for all the cells and the population curve fit to a higher staining and lower staining population, and cells assigned to the population to which they most statistically are likely to belong in view of a statistical analysis of the respective population distributions. In some embodiments, the CD4- cells stain two to three-fold less intensely than the CD4⁺ cells.
As used herein, the term "CD25" refers to the alpha subunit of interleukin-2 receptor, a single-chain glycoprotein with a molecular weight of 55 kD. Following the activation of T cells with antigen or mitogen in the presence of the monokine interleukin-1, interleukin 2 (IL-2) is rapidly synthesized and secreted. In response to this, a subpopulation of T cells expresses high affinity receptors for IL-2. These cells proliferate, expanding the T cell population which is capable of mediating helper, suppressor and cytotoxic functions. IL-2 receptor is not uniquely found on T cells. "CD25^{hi}" refers to cells which stain brightly when contacted with labeled anti-CD25 antibody, "CD25⁺" refers to cells which stain less brightly when contacted with labeled anti-CD25 antibody, and "CD25^{lo/-}" refers to cells which are of a type which stains the least brightly dull or null when contacted with a labeled CD25 antibody. Generally, the cells are distinguished according to their CD25 expression levels based upon differences in staining intensity as is known to one of ordinary skill in the art. In some embodiments, the cut off for designating a cell as a CD25 expression category hi, +, lo, or - cell can be set in terms of the fluorescent intensity distribution observed for all the cells. Generally, cells in the top 2, 3, 4, or 5% of staining intensity are designated "hi", with those falling in the top half of the population categorized as being "+". Those cells falling below 50%, of fluorescence intensity are designated as CD25^{lo} cells and below 5% as CD25- cells.
As used herein, the term "Foxp3" refers to the nuclear protein Foxp3 believed to act as a transcription factor (Hori et al., 2003; Yasayko, J. E. et al., Nat. Genet. 27:68-73 (2001); Fontenot, J. D. et al., Nat. Immunol. 4:330-336 (2003); Khattri, R. et al., Nat. Immunol. 4:337-342 (2003)). As being intra cellularly located, Foxp3 expression may be assessed by intracellular flow cytometry using a labeled anti-Foxp3 antibody (eBioscience inc or BD biosciences).
According to the invention, the Tr1 cells are capable of secreting high levels of IL-10 and intermediate levels of TGF-β upon activation. Tr1 cells may be characterized, in part, by their unique cytokine profile: they produce high levels of IL-10, intermediate levels of TGF-β and intermediate levels of IFN-γ, but little or no IL-4 or IL-2. The cytokine production is typically evaluated in cultures of cells after activation with polyclonal activators of T lymphocytes such as anti-CD3 + anti-CD28 antibodies or Interleukin-2, PMA + ionomycin. Alternatively, the cytokine production is evaluated in cultures of cells after activation with the specific T-cell antigen presented by antigen presenting cells. High levels of IL-10 correspond to at least about 500 pg/ml, typically greater than about 1, 2, 4, 6, 8, 10, 12, 14, 16, 18, or 20 thousand pg/ml or more. Intermediate levels of TGF-β correspond to at least about 100 pg/ml, typically greater than about 200, 300, 400, 600, 800, or 1000 pg/ml or more. Intermediate levels of IFN-γ correspond to concentrations comprised between 0.1 pg/ml and at least 400 pg/ml, typically greater than about 600, 800, 1000, 1200, 1400, 1600, 1800, or 2000 pg/ml or more. Little or no IL-4 or IL-2 corresponds to less than about 500 pg/ml, preferably less than about 250, 100, 75, or 50 pg/ml, or less.
Tr1 cells can be operationally characterized by cell surface markers. These cell surface markers can be recognized by reagents that specifically bind to the cell surface markers. For example, proteins, carbohydrates, or lipids on the surface of Tr1 cells can be immunologically recognized by antibodies specific for the particular protein or carbohydrate (for use of antibodies to markers, see, Harlow, Using Antibodies: A Laboratory Manual (Cold Spring Harbor Press, Cold Spring Harbor, N.Y., 1999); see also, EXAMPLES). The set of markers present on the surface of Tr1 cells and absent from the surface of these cells is characteristic for Tr1 cells. Therefore, Tr1 cells can be selected by positive and negative selection using cell surface markers. A reagent that binds to a cell surface marker expressed by a Tr1 cell, a "positive marker", can be used for the positive selection of Tr1 cells (i.e., retaining cells that express the cell surface marker). Conversely, negative selection relies on that fact that certain cell surface markers are not expressed by Tr1 cells. Therefore, a "negative marker" (i.e., a marker not present on the cell surface of Tr1 cells) can be used for the elimination of those cells in the population that are not Tr1 cells by the removal of cells that bind to the reagent specific for the negative marker.
In one embodiment, discrimination between cells based upon the detected expression of cell surface markers occurs by comparing the expression of a cell surface marker with the mean expression by a control population of cells. For example, the expression of a marker on a Tr1 cell can be compared to the mean expression of the same marker on other cells derived from the same sample as the Tr1 cell. Other methods of discriminating among cells by marker expression include gating cells by flow cytometry using a combination of reagents (see, Givan A, Flow Cytometry: First Principles, (Wiley-Liss, New York, 1992); Owens M A & Loken M R., Flow Cytometry: Principles for Clinical Laboratory Practice, (Wiley-Liss, New York, 1995)).
By a "combination of reagents" is meant at least two reagents that bind to cell surface markers either present (positive marker) or not present (negative marker) on the surfaces of Tr1 cells, or that bind to a combination of positive and negative markers. For example, the use of a combination of antibodies specific for Tr1 cell surface markers results in isolation and/or enrichment of Tr1 cells from a variety of samples/tissues.
By selecting for phenotypic characteristics among the cells obtained from the sample, antibodies that recognize species-specific varieties of markers are used to enrich for, and select Tr1 cells. "Enriched", as in an enriched population of cells, can be defined based upon the increased number of cells having a particular marker in a fractionated set of cells as compared with the number of cells having the marker in the unfractionated set of cells. In particular embodiments, the Tr1 cells are enriched from a population of cells using reagents that bind cell surface markers specific for Tr1 cells and separating these cells using cell sorting assays such as fluorescence-activated cell sorting (FACS), solid-phase magnetic beads, etc. In some embodiments, combinations of methods to sort the cells can be used, e.g., magnetic selection, followed by FACS. To enhance enrichment, positive selection is combined with negative selection for Tr1 cell isolation using cell surface markers. It is intended that isolation/enrichment of Tr1 cells using cell surface markers can be performed in any order. Therefore, a positive selection step may immediately precede a negative selection step, or vice versa. It is also contemplated that isolation/enrichment be performed by grouping the positive selection and negative selection steps. Therefore, isolation/enrichment is done by first performing the positive selection steps of the method, followed by performing the negative selection steps of the method, or vice versa.
An "anti-X antibody" or "X antibody" according to the invention is an antibody which can specifically bind to X. For instance, the anti-CD127 antibody or CD127 antibody is capable of binding CD127. The antibodies for use according to the invention include, but are not limited to, recombinant antibodies, polyclonal antibodies, monoclonal antibodies, chimeric antibodies, human monoclonal antibodies, humanized or primatized monoclonal antibodies, and antibody fragments. A great many lymphocyte biomarker specific antibodies are commercially available. These include anti-CD127, anti-CD4, anti-CD62L and anti-CD25 antibodies. "Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding. An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively. Antibodies exist, e.g., as intact immunoglobulins or as a number of well-characterized fragments produced by digestion with various peptidases. Thus, for example, pepsin digests an antibody below the disulfide linkages in the hinge region to produce F(ab)'2, a dimer of Fab which itself is a light chain joined to VH-CH1 by a disulfide bond. The F(ab)'2 may be reduced under mild conditions to break the disulfide linkage in the hinge region, thereby converting the F(ab)'2 dimer into an Fab' monomer. The Fab' monomer is essentially Fab with part of the hinge region (see Fundamental Immunology (Paul ed., 3d ed. 1993). While various antibody fragments are defined in terms of the digestion of an intact antibody, one of skill will appreciate that such fragments may be synthesized de novo either chemically or by using recombinant DNA methodology. Thus, the term antibody, as used herein, also includes antibody fragments either produced by the modification of whole antibodies, or those synthesized de novo using recombinant DNA methodologies (e.g., single chain Fv) or those identified using phage display libraries (see, e.g., McCafferty et al., Nature 348:552-554 (1990)). In some embodiments, a high affinity ligand of a target may be used in place of the antibody. The phrase "specifically (or selectively) binds" to an antibody or "specifically (or selectively) immunoreactive with," when referring to a protein or peptide, refers to a binding reaction that is determinative of the presence of the protein, often in a heterogeneous population of proteins and other biologics. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules. Preferably a "label" or a "detectable moiety" is covalently or noncovalently attached to the antibody. A label may be detectable by spectroscopic, photochemical, biochemical, immunochemical, chemical, or other physical means. Particularly useful labels are fluorescent dyes. Methods of attaching labels to antibodies are well known to those of ordinary skill in the art. Particularly preferred labels are those which are attached to the antibody by a linker which can be readily cleaved or separated or subject to hydrolysis by contact with a predetermined enzyme under physiological conditions. The antibody may also be conjugated with a magnetic particle, such as a paramagnetic microbead (Miltenyi Biotec, Germany). An activated T cell bound by a magnetically labeled antibody may be isolated using techniques including, but not limited to, magnetic cell sorting. Suitably labeled antibodies to CD127, CD62L, CD4 and CD25, as well as many other cluster of differentiation, are commercially available and known to one of ordinary skill in the art. The antibody may be labeled before or after contact with the sample or before or after contact with the CD. The CD antibody may be labeled by contacting with a labeled antibody which binds to the CD-antibody. As used herein, the term "CD" or "cluster of differentiation" or "common determinant" refers to cell surface molecules recognized by antibodies.

According to the invention, a Tr1 cell population can be identified by detecting the cell surface expression of CD4, CD127 and CD62L markers on a cell population: the cells expressing CD4 (i.e. being CD4⁺), and expressing a low level of CD127 and CD62L (i.e. being CD127^{lo/-} and CD62L^{lo/-}) correspond to Tr1 cells.
According to the invention, a resting Tr1 cell population can be identified by detecting the cell surface expression of CD4, CD25, and at least one of CD127 and CD62L markers on a cell population: the cells expressing CD4 (i.e. being CD4⁺) and expressing a low level of at least one of CD127 and CD62L (i.e. being CD127^{lo/-} and/or CD62L^{lo/-}), preferably a low level of CD127 and CD62L, and not expressing CD25 (i.e. being CD25⁻) correspond to the resting Tr1 cells.
In one embodiment of the invention, the marker Foxp3 is also assessed and the cells expressing CD4 (i.e. being CD4⁺) and expressing a low level of at least one of CD127 and CD62L (i.e. being CD127^{lo/-} and/or CD62L^{lo/-}), preferably a low level of CD127 and CD62L, and not expressing CD25 (i.e. being CD25⁻), and further not expressing Foxp3 (i.e. being Foxp3⁻) correspond to the resting Tr1 cells.
According to the invention, an activated Tr1 cell population can be identified by detecting the cell surface expression of CD4, CD25, CD127 and CD62L markers on a cell population: the cells expressing CD4 and CD25 (i.e. being CD4⁺CD25⁺) and expressing a low level of CD127 and CD62L (i.e. being CD127^{lo/-} and CD62L^{lo/-}) are the activated Tr1 cells.
In one embodiment, the marker Foxp3 is also assessed and the cells expressing CD4 and CD25 (i.e. being CD4⁺CD25⁺) and expressing a low level of CD127 and CD62L (i.e. being CD127^{lo/-} and/or CD62L^{lo/-}), and expressing Foxp3 (i.e. being Foxp3⁺) are the activated Tr1 cells.
Preferred reagents that bind to said markers (CD4, CD25, CD127, CD62L and Foxp3) for identifying/selecting said Tr1 cells are antibodies. In a further embodiment, the antibodies are conjugated to a fluorochrome or magnetic particle. In another embodiment, the cell selection is performed by flow cytometry, fluorescence activated cell sorting, magnetic selection, affinity chromatography or panning or combinations thereof.

Another object of the invention is an isolated population of Tr1 cells obtained by the identification method as described here above and then by its isolation.
Another object of the invention is an isolated population of resting Tr1 cells obtained by the identification method as described here above and then by its isolation. Another object of the invention is an isolated population of activated Tr1 cells obtained by the identification method as described here above and then by its isolation.
Another object of the invention is an isolated population of resting and activated Tr1 cells obtained by the identification method as described here above and then by its isolation.
"Isolated" refers to a cell or a cell population that is removed from its natural environment (such as the peripheral blood) and that is isolated or separated, and is at least about 75% free, 80% free, 85% free and preferably about 90%, 95%, 96%, 97%, 98%, 99% free, from other cells with which it is naturally present, but which lack the cell surface markers based on which the cells were isolated.
Isolation methods of a cell population are well known in the art and include, but are not limited to, cell sorting by flow cytometry, magnetic selection, affinity chromatography, panning or combinations thereof.

Another object of the invention is a pharmaceutical composition comprising or consisting of the isolated population of resting Tr1 cells and/or activated Tr1 cells as described here above.
In one embodiment, said pharmaceutical composition comprises a pharmaceutically acceptable carrier.
The pharmaceutically acceptable carriers useful herein are conventional. Remington's Pharmaceutical Sciences 16th edition, Osol, A. Ed. (1980) describes compositions and formulations suitable for pharmaceutical delivery of the composition of the present invention. In general, the nature of the carrier will depend on the mode of administration being employed. For instance, parenteral formulations usually comprise injectable fluids that include pharmaceutically and physiologically acceptable fluids such as water, physiological saline, balanced salt solutions, aqueous dextrose, sesame oil, glycerol, ethanol, combinations thereof, or the like, as vehicle. The carrier and composition can be sterile, and the formulation suits the mode of administration. In addition to biological neutral carriers, pharmaceutical compositions to be administrated can contain minor amounts of non-toxic auxiliary substances, such as wetting or emulsifying agents, preservatives, and pH buffering agents and the like, for example sodium acetate or sorbitan monolaurate. The composition can be a liquid solution, suspension, emulsion.

Another object of the invention is a method for enriching a cell population in Tr1 cells, comprising:
- identifying the cells expressing CD4 and a low level of CD127 and CD62L,
- selecting said cells,
- thereby obtaining a cell population enriched in Tr1 cells.

Another object of the invention is a method for enriching a cell population in resting Tr1 cells, comprising:
- identifying the cells expressing CD4 and a low level of at least one of CD127 and CD62L, preferably a low level of CD127 and CD62L, and not expressing CD25,
- selecting said cells,
- thereby obtaining a cell population enriched in resting Tr1 cells.
Another object of the invention is a method for enriching a cell population in activated Tr1 cells, comprising:
- identifying the cells expressing CD4, CD25 and a low level of CD127 and CD62L,
- selecting said cells and isolating them,
- thereby obtaining a cell population enriched in activated Tr1 cells.

Another object of the invention is an enriched population of Tr1 cells obtained according to the method as described here above.
Another object of the invention is an enriched population of resting Tr1 cells obtained according to the method as described here above.
Another object of the invention is an enriched population of activated Tr1 cells obtained according to the method as described here above.

Preferred reagents that bind to said markers (CD4, CD25, CD127 and CD62L) for selecting/enriching said cells are antibodies. In a further embodiment, the antibodies are conjugated to a fluorochrome or magnetic particle. In another embodiment, the cell selection is performed by flow cytometry, fluorescence activated cell sorting, magnetic selection, affinity chromatography or panning or combinations thereof.

A Tr1 cell enriched composition/population is one in which the percentage of Tr1 cells is higher than the percentage of Tr1 cells in the originally obtained population of cells. Although possible, an enriched population of Tr1 cells need not contain a homogenous population of Tr1 cells. In particular embodiments, at least about 50%, about 55%, about 60%, about 65%, about 70%, about 75%, about 80%, about 85%, about 90%, about 95%, about 98%, or about 99% of said cells of the composition are Tr1 cells. In another embodiment, the percentage of Tr1 cells in the enriched composition/population is at least twice, 5 times, 10 times, 20 times, 30 times, 40 times, 50 times, 60 times, 70 times, 80 times, 90 times, 100 times the percentage of Tr1 cells before enrichment. As used herein, the term "about" preceding a figure means more or less 10% of the value of the figure.
Any cellular source that contains T cells can be used to isolate/enrich Tr1 cells. Useful sources include, but are not limited to, peripheral blood, synovial fluid, spleen, thymus, lymph nodes, bone marrow, Peyer's patches, and tonsils.
Enrichment methods are variable based on the level of enrichment associated with each step of the enrichment process. The level of enrichment and percent purity of the Tr1 cells will depend on many factors including, but not limited to, the donor, the cell/tissue source and the disease state of the donor. In particular embodiments, the Tr1 cells are enriched at least about 2-fold, about 5-fold, about 10-fold, about 15-fold, about 20-fold, about 25-fold, about 30-fold, about 35-fold, about 40-fold, about 50-fold, about 55-fold, about 60-fold, about 65-fold, about 70-fold, about 75-fold, about 80-fold, about 85-fold, about 90-fold, about 95-fold, about 100-fold, about 105-fold, about 110-fold, about 115-fold, about 120-fold, about 130-fold, about 140-fold, about 150-fold, or about 200-fold.

Methods for separating, isolating, or selecting cells include, but are not limited to magnetic separation using antibody-coated magnetic beads (Schwartz, et al, U.S. Pat. No. 5,759,793) and affinity chromatography or "panning" using antibody attached to a solid matrix (e.g. a plate). Further techniques providing accurate separation include fluorescence-activated cell sorters (FACS), which can have varying degrees of sophistication, such as having multiple color channels, low angle and obtuse light scattering detecting channels, or impedance channels. Dead cells can be eliminated by selection with dyes associated with dead cells e.g., (propidium iodide, LDS). Red blood cells can be removed by, for example, elutriation, hemolysis, or Ficoll-Paque gradients. Any technique can be employed that is not unduly detrimental to the viability of the selected cells.

Conveniently, antibodies can be conjugated with labels for a number of different purposes: e.g., magnetic beads to allow for ease of separation of Tr1 cells; biotin, which binds with high affinity to avidin or streptavidin; fluorochromes, which can be used with a fluorescence activated cell sorter; haptens; and the like. Multi-color analyses can be employed with FACS or in a combination of immunomagnetic separation and flow cytometry. Multi-color analysis is of interest for the separation of cells based on multiple surface antigens: e.g., non-CD4⁺ immune cell markers (CD8, CD14, CD16, CD19, CD36, CD56, CD123, TCRgamma/delta and glycophorin A), CD4, CD25, CD127 and CD62L. Fluorochromes which find use in a multi-color analysis include, but are not limited to, phycobiliproteins, e.g. phycoerythrin and allophycocyanins; fluorescein, and Texas red.
Magnetic separation is a process used to selectively retain magnetic materials within a vessel, such as a centrifuge tube or column, in a magnetic field gradient. Tr1 cells can be magnetically labeled by binding magnetic particles to the surface of the cells through specific interactions, including immuno-affinity interactions. The suspension, containing the Tr1 cells within a suitable vessel, is then exposed to magnetic field gradients of sufficient strength to separate the Tr1 cells from other cells in the suspension. The vessel can then be washed with a suitable fluid to remove the unlabeled cells, resulting in a purified suspension of Tr1 cells.
The majority of magnetic labeling systems use superparamagnetic particles with monoclonal antibodies or streptavidin covalently bound to their surface. In cell separation applications, these particles can be used for either positive selection, where the cells of interest are magnetically labeled and retained, or negative selection where the majority of undesired cells are magnetically labeled and retained. The diameter of the particle used varies widely from about 50-100 nm for MACS particles (Miltenyi Biotec) and StemSep(TM) colloid (StemCell Technologies), through 150-450 nm for EasySep(R) (StemCell Technologies) and Imag particles (BD Biosciences), up to 4.2 µm for Dynabeads (Dynal Biotech). The type of particle used is influenced by the magnet technology employed to separate the labeled cells.
There are two important classes of magnetic separation technologies, both of which, for convenience and for practical reasons, use permanent magnets as opposed to electromagnets. The first class is column-based high-gradient-magnetic-field separation technology that uses small, weakly magnetic particles to label the targets of interest, and separates these targets in a column filled with a magnetizable matrix. Very high gradients are generated close to the surface of the matrix elements when a magnetic field is applied to the column. The high gradients are necessary to separate targets labeled with these relatively weakly magnetic particles. The second class is tube-based technology that uses more strongly magnetic particles to label the targets of interest. These targets of interest are then separated within a centrifuge-type tube by magnetic field gradients generated by a magnet outside the tube. This method has the advantage that it does not rely on a magnetizable matrix to generate the gradients; and, therefore does not require an expensive disposable column or a reusable column with an inconvenient cleaning and decontamination procedure.
Once placed within the magnet, targeted cells migrate toward the region or regions of highest magnetic field strength and are retained within the magnetic field while the unlabeled cells are drawn off. The targeted cells can then be collected and used after removal from the magnetic field. In the event that negative selection is required, the unlabeled cells are retained and can be utilized for a variety of applications.
FACS permits the separation of sub-populations of cells on the basis of their light scatter properties as they pass through a laser beam. The forward light scatter (FALS) is related to cell size, and the right angle light scatter, also known as side scatter characteristic (SSC) is related to cell density, cellular content and nucleo-cytoplasmic ratio, i.e. cell complexity. Since cells can be labeled with fluorescent-conjugated antibodies, they can further be characterized by antibody (fluorescence) intensity.
In particular embodiments, the Tr1 cells of the present invention, are isolated using immuno-magnetic chromatography. For example, an anti-CD4 antibody is attached to magnetic beads. These antibody-labeled magnetic beads are used as the basis for the affinity purification. The antibody-labeled fraction of T cells is applied to the magnetic affinity column. The non-adherent cells are discarded and the adherent cells are eluted from the magnetic column by removal of the magnetic field. In another embodiment, the cells are first labeled with an antibody (e.g., anti-CD4) and then labeled with a secondary antibody carrying a magnetic bead or sphere.
In another embodiment, a secondary antibody immunoreactive with a Tr1 cell can be used to enrich the population of Tr1 cells. The use of a secondary antibody is generally known in the art. Typically, secondary antibodies are antibodies immunoreactive with the constant regions of the first antibody. Preferred secondary antibodies include anti-rabbit, anti-mouse, anti-rat, anti-goat, and anti-horse immunoglobulins and are available commercially. Commercially available kits provide secondary antibodies conjugated to labeling agents such as, but limited to, magnetic particles and fluorochromes.

In one embodiment of the invention, the method for enriching a population of Tr1 cells, resting Tr1 cells or activated Tr1 cells may comprise:
- contacting the cell population with at least one reagents which binds to a marker of non-CD4⁺ cells, thereby depleting non-CD4⁺ cells,
- identifying Tr1 cells, resting Tr1 cells or activated Tr1 cells as described here above and selecting and isolating them.
Examples of markers that bind to non-CD4⁺ cells include, but are not limited to, CD8, CD14, CD16, CD19, CD36, CD56, CD123, and glycophorin A.
Preferred reagents that bind to said markers are antibodies. In a further embodiment, the antibodies are conjugated to a fluorochrome or magnetic particle. In another embodiment, the cell selection is performed by flow cytometry, fluorescence activated cell sorting, magnetic selection, affinity chromatography or panning or combinations thereof.

In one embodiment of the invention, the Tr1 cells isolated according to the present invention may be further expanded by the in vitro method described in WO2006/108882. Said method comprises:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the Tr1 cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.
Examples of factors which interact with the above-mentioned cell surface proteins include:
- an anti-CD3 monoclonal antibody or a modified anti-CD3 antibody, wherein the anti-CD3 intracytoplasmic domain of the CD3 heavy chain is replaced with a transmembrane domain,
- an anti-CD28 antibody or fragment thereof or the CD80 or CD86 protein,
- the IL-2 secreted by the feeder cells,
- the CD58 protein,
- an interleukin selected from the group comprising IL-4 and IL-13.
In an embodiment of the invention, the Tr1 cells thus obtained may be cloned by using conventional methods for cloning T cells.
In an embodiment of the present invention, the Tr1 cells thus obtained or the Tr1 cell clones may be frozen to be stored.

Another object of the invention is a method for depleting a cell population in Tr1 cells, comprising:
- identifying the cells expressing CD4 and a low level of CD127 and CD62L,
- depleting said cells,
- thereby obtaining/isolating a cell population depleted in Tr1 cells.
Another object of the invention is a method for depleting a cell population in resting Tr1 cells, comprising:
- identifying the cells expressing CD4 and a low level of at least one of CD127 and CD62L, preferably a low level of CD27 and CD62L, and not expressing CD25,
- depleting said cells,
- thereby obtaining/isolating a cell population depleted in resting Tr1 cells.
Another object of the invention is a method for depleting a cell population in activated Tr1 cells, comprising:
- identifying the cells expressing CD4, CD25 and a low level of CD127 and CD62L,
- depleting said cells,
- thereby obtaining/isolating a cell population depleted in activated Tr1 cells.

A Tr1 cell-depleted composition/population is one in which the percentage of Tr1 cells is lower than the percentage of Tr1 cells in the originally obtained population of cells.
Another object of the invention is a Tr1 cells-depleted cell population obtained according to the method as described here above.
Another object of the invention is a resting Tr1 cells-depleted cell population obtained according to the method as described here above.
Another object of the invention is an activated Tr1 cells-depleted cell obtained according to the method as described here above.

As explained here above, methods for isolating cells include, but are not limited to, magnetic separation using antibody-coated magnetic beads, affinity chromatography or "panning" using antibody attached to a solid matrix (e.g. a plate) and fluorescence-activated cell sorters (FACS). Instead of being selected, the Tr1 cells are depleted.
In one embodiments, the percentage of Tr1 cells in the depleted composition/population is at most 0.75 times, 0.7 times, 0.6 times, 0.5 times, 0.4 times, 0.3 times, 0.25 times, 0.2 times, 0.15 times, 0.1 times the percentage of Tr1 cells before depletion.

Another object of the invention is a pharmaceutical composition comprising or consisting of a resting and/or activated Tr1 cells-depleted cell population and a pharmaceutically acceptable carrier.

Another object of the invention is a kit for identifying or isolating a Tr1 cell population, a resting Tr1 cell population or an activated Tr1 cell population, or for enriching or depleting a cell population in Tr1 cells, resting Tr1 cells or activated Tr1 cells, said kit comprising reagents for detecting the cell surface expression of CD4, CD25, CD127 and CD62L.
Preferably, said reagents are antibodies. In another embodiment, these antibodies are conjugated to a fluorochrome or magnetic particle.
In another embodiment, said kit further comprises a reagent for detecting Foxp3 expression. Preferably, said reagent is an antibody. More preferably, said antibody is conjugated to a fluorochrome.

Another object of the present invention is a method for inhibiting an immune response in a subject in need thereof, comprising the administration of an effective amount of the isolated or enriched Tr1 cells, resting Tr1 cells or activated Tr1 cells according to the invention to the subject.
Another object is a method for inhibiting an immune response in a subject in need thereof, comprising:
- isolating resting Tr1 cells from a biological sample according to the method as described here above,
- expanding said Tr1 cells,
- administrating the expanded Tr1 cells to the subject.

Accordingly, the expanded Tr1 cells administrated to the subject inhibit an undesired immune response by their suppressive activity, thereby allowing the treatment of conditions associated with undesired immune response.

One advantage of isolating resting Tr1 cells in view of cell therapy is that the cell population to be administrated is pure. Another advantage of using resting Tr1 cells is their efficiency. In fact, it is known by the skilled person in the art that activating an already activated T cell population would induce cell mortality in vitro, leading to less efficiency in vivo. Still another advantage of using a pure Tr1 cell population lies in time effectiveness and cost effectiveness. In fact, expanding in vitro this population in a number sufficient for cell therapy needs only about 2 weeks.

In one embodiment of the invention, the resting Tr1 cells may be obtained from blood, such as peripheral blood or umbilical cord blood, or from tissue biopsy such as lymph node biopsy, intestinal or synovial biopsies or mucosal tissue biopsy, or from bronchoalveolar lavage or a cerebrospinal fluid.
In another embodiment, the resting Tr1 cells are autologous or allogeneic. The term "allogeneic cells" as used herein refers to cells isolated from one subject (the donor) and infused in another (the recipient or host). The term "autologous cells" as used herein refers to cells that are isolated and infused back into the same subject.

Methods for expanding the resting Tr1 cell population are well known in the art.
In one embodiment of the invention, the resting Tr1 cells are expanded in vitro as described in WO2006/108882. Said method comprises:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the Tr1 cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.
Examples of factors which interact with the above-mentioned cell surface proteins include:
- an anti-CD3 monoclonal antibody or a modified anti-CD3 antibody, wherein the anti-CD3 intracytoplasmic domain of the CD3 heavy chain is replaced with a transmembrane domain,
- the CD80 or CD86 protein,
- the IL-2 secreted by the feeder cells,
- the CD58 protein,
- an interleukin selected from the group comprising IL-4 and IL-13.
An anti-CD3 monoclonal antibody can be used to activate a population of T cells via the TCR/CD3 complex, advantageously a modified anti-CD3 antibody, wherein the modification of the anti-CD3 antibody consists in the replacement of the intracytoplasmic domain with a transmembrane domain, such that said modified anti-CD3 antibody anchors to the cellular membrane of the feeder cells and interacts with the CD3/TCR protein complex of the T cells. The factor interacting with the CD28 protein present at the surface of the antigen-specific Tr1 cells and which is expressed by the feeder cells, may be an anti-CD28 monoclonal antibody or a fragment thereof capable of crosslinking the CD28 molecule; in such a case, modification of the anti-CD28 monoclonal antibody can be envisaged by adding a transmembrane domain in order that it anchors to the cell surface of the feeder cells. Preferably, the natural ligand for CD28 is employed instead of the anti-CD28 monoclonal antibody, that is to say for example a member of the B7 family of proteins, such as B7-1(CD80) and B7-2 (CD86) proteins.
The factor expressed by the feeder cells which interacts with CD2 may be an anti-CD2 monoclonal antibody or a fragment thereof capable of crosslinking the CD2 molecule; modification of the anti-CD2 monoclonal antibody can be envisaged by adding a transmembrane domain for anchoring to the cell surface of the feeder cells.

Preferably, the natural ligand for CD2 is employed instead of the anti-CD2 monoclonal antibody, that is to say the CD58 protein.
In addition to the factors which are anchored to the cell membrane of the feeder cells, factors which are secreted, such as interleukins, are also required for expansion of the antigen-specific Tr1 cell population. Among these interleukins are the IL-2, which interacts with the IL-2 receptor present at the surface of the antigen-specific Tr1 cells, and either the IL-4 or the IL-13, which interacts with the IL-4 receptor of the antigen-specific Tr1 cells.

In another embodiment of the invention, the resting Tr1 cells are expanded by culturing the Tr1 cells with anti-CD3/28 beads in the presence of cytokines such as IL-2, IL-4, IL-13 and/or IL-15. An example of said kit for expanding the resting Tr1 cells is the Dynabeads® kit commercialized by Invitrogen (Cat N°111-41D).

In another embodiment of the invention, the resting Tr1 cells are expanded by culturing the Tr1 cells with autologous PBL, leucocytes, PBMC, antigen presenting cells or artificial antigen presenting cells expressing an MHC class II such as L cells, in the presence of an antigen.

In another embodiment of the invention, the resting Tr1 cells are expanded by culturing the Tr1 cells with mitogen such as PHA in the presence of cytokine such as IL-2.

In an embodiment of the invention, the Tr1 cells thus expanded may be cloned by using conventional methods for cloning T cells.
In an embodiment of the present invention, the Tr1 cells thus expanded or the Tr1 cell clones may be frozen to be stored.

In one embodiment of the invention, the expanded Tr1 cells are specific of an antigen or are specific of multiple antigens.
Examples of antigen to which the expanded Tr1 cells may be specific include, but are not limited to, auto-antigens; food antigen from common human diet; inflammatory antigens such as multiple sclerosis-associated antigens or joint-associated antigens; and allergens.
The term "food antigen from common human diet" refers to an immunogenic peptide, which comes from foodstuffs common for humans, such as food antigens of the following non-limiting list: bovine antigens such as lipocalin, Ca-binding S100, alpha-lactalbumin, lactoglobulins such as beta-lactoglobulin, bovine serum albumin, caseins. Food-antigens may also be atlantic salmon antigens such as parvalbumin, chicken antigens such as ovomucoid, ovalbumin, Ag22, conalbumin, lysozyme or chicken serum albumin, peanuts, shrimp antigens such as tropomyosin, wheat antigens such as agglutinin or gliadin, celery antigens such as celery profilin, carrot antigens such as carrot profilin, apple antigens such as thaumatin, apple lipid transfer protein, apple profilin, pear antigens such as pear profilin, isoflavone reductase, avocado antigens such as endochitinase, apricot antigens such as apricot lipid transfer protein, peach antigens such as peach lipid transfer protein or peach profilin, soybean antigens such as HPS, soybean profilin or (SAM22) PR-I0 prot.
The term "auto-antigen" refers to an immunogenic peptide derived from a protein of said individual. It may be, by way of example, an auto-antigen of the following non-limiting list: acetylcholine receptor, actin, adenine nucleotide translocator, adrenoreceptor, aromatic L-amino acid decarboxylase, asialoglycoprotein receptor, bactericidal/permeability increasing protein (BPi), calcium sensing receptor, cholesterol side chain cleavage enzyme, collagen type IV-chain, cytochrome P450 2D6, desmin, desmoglein-1, desmoglein-3, F-actin, GM-gangliosides, glutamate decarboxylase, glutamate receptor, H/K ATPase, 17- -hydroxylase, 21-hydroxylase, IA-2 (ICAS12), insulin, insulin receptor, intrinsic factor type 1, leucocyte function antigen 1, myelin associated glycoprotein, myelin basic protein, myelin oligodendrocyte protein, myosin, P80-coilin, pyruvate dehydrogenase complex E2 (PDC-E2), sodium iodide symporter, SOX-10, thyroid and eye muscle shared protein, thyroglobulin, thyroid peroxidase, thyrotropin receptor, tissue transglutaminase, transcription coactivator p75, tryptophan hydroxylase, tyrosinase, tyrosine hydroxylase, ACTH, aminoacyl-tRNA-hystidyl synthetase, cardiolipin, carbonic anhydrase II, centromere associated proteins, DNA-dependent nucleosome-stimulated ATPase, fibrillarin, fibronectin, glucose 6 phosphate isomerase, beta 2-glycoprotein I, golgin (95, 97, 160, 180), heat shock proteins, hemidesmosomal protein 180, histone H2A, H2B, keratin, IgE receptor, Ku-DNA protein kinase, Kunucleoprotein, La phosphoprotein, myeloperoxidase, proteinase 3, RNA polymerase I-III, signal recognition protein, topoisomerase I, tubulin, vimentin, myelin associated oligodendrocyte basic protein (MOBP), proteolipid protein, oligodendrocyte specific protein (OSP/Claudin 11), cyclic nucleotide 3'phosphodiesterase (CNPase), BP antigen 1 (BPAG1-e), transaldolase (TAL), human mitochondrial autoantigens PDC-E2 (Novo 1 and 2), OGDC-E2 (Novo 3), and BCOADC-E2 (Novo 4), bullous pemphigoid (BP)180, laminin 5 (LN5), DEAD-box protein 48 (DDX48) or insulinoma-associated antigen-2.
The term "multiple sclerosis-associated antigen" refers to myelin basic protein (MBP). myelin associated glycoprotein (MAG), myelin oligodendrocyte protein (MOG), proteolipid protein (PLP), oligodendrocyte myelin oligoprotein (OMGP). myelin associated oligodendrocyte basic protein (MOBP), oligodendrocyte specific protein (OSP/Claudinl 1), heat shock proteins, oligodendrocyte specific proteins (OSP), NOGO A, glycoprotein Po, peripheral myelin protein 22 (PMP22), 2'3'-cyclic nucleotide 3'-phosphodiesterase (CNPase), fragments, variants and mixtures thereof. The term "joint-associated antigen" refers to citrulline-substituted cyclic and linear filaggrin peptides, collagen type II peptides, human cartilage glycoprotein 39 (HCgp39) peptides, HSP, heterogenous nuclear ribonucleoprotein (hnRNP) A2 peptides, hnRNP Bl, hnRNP D, Ro60/52, HSP60, 65, 70 and 90, BiP, keratin, vimentin, fibrinogen, collagen type I, III, IV and V peptides, annexin V, Glucose 6 phosphate isomerase (GPI), acetyl-calpastatin, pyruvate dehydrogenase (PDH), aldolase, topoisomerase I, snRNP, PARP, Scl-70, Scl-100, phospholipid antigen including anionic cardiolipin and phosphatidylserine, neutrally charged phosphatidylethanolamine and phosphatidylcholine, matrix metalloproteinase, fibrillin, aggreccan.
The term "allergen" refers to an inhaled allergen, an ingested allergen or a contact allergen. Examples of allergens include, but are not limited to, inhaled allergens derived from pollens (Cup, Jun), house dust mites (Der, Gly, Tyr, Lep), dog, cat and rodents (Can, Fel, Mus, Rat). Examples of contact allergens include, but are not limited to, heavy metals (such as nickel, chrome, gold), latex, haptens such as halothane, hydralazine.

In one embodiment of the invention, the expanded Tr1 cells may be formulated for parenteral, intramuscular, intra-tissular, intravenous, or intra-peritoneal injection, intranasal inhalation, lung inhalation, intradermal, or intra-articular injection. Preferably, the expanded Tr1 cells may be administrated by intramuscular, intraperitoneal or intravenous injection, or by direct injection into the lymph nodes of the patient, more preferably by intravenous injection.

Another object of the invention is a pharmaceutical composition comprising or consisting of the expanded Tr1 cells, which are a mixture of resting Tr1 cells and activated Tr1 cells and a pharmaceutically acceptable carrier.
Another object of the invention is a pharmaceutical composition comprising a mixture of resting Tr1 cells and activated Tr1 cells, wherein the mixture is obtained by expanding in vitro an isolated resting Tr1 cell population obtained by the method described here above.
Accordingly, the pharmaceutical composition comprises resting Tr1 cells having the following phenotype CD4⁺CD25-CD127^{lo/-}CD62L^{lo/-} and activated Tr1 cells having the following phenotype CD4⁺CD25⁺CD127^{lo/-}CD62L^{lo/-}.
The phenotype of the expanded Tr1 cells to be administrated to the subject in need thereof can be analyzed by flow cytometry.
At the end of the expansion step, the expanded Tr1 cells remain in two states: a resting state or an activated state. Without willing to be bound to a theory, the inventors suggest that a composition comprising a mixture of resting Tr1 cells and activated Tr1 cells is of interest as the activated Tr1 cells will be able to suppress inflammation directly after administration, while the resting Tr1 cells will need to be activated in situ to proliferate in vivo and exert their suppressive activity at a long term.

In one embodiment, the subject has undergone or is undergoing transplantation such as a bone marrow transplantation or an organ transplantation.

In another embodiment, the subject has an infection, such as a microbial infection, an RSV infection, an allergy.
Another object of the present invention is a method for preventing or treating an inflammatory condition in a subject in need thereof, comprising the administration of an effective amount of the isolated or enriched Tr1 cells, resting Tr1 cells or activated Tr1 cells according to the invention to the subject.
Another object of the present invention is a method for preventing or treating an autoimmune condition in a subject in need thereof, comprising the administration of an effective amount of the isolated or enriched Tr1 cells, resting Tr1 cells or activated Tr1 cells according to the invention to the subject.

In one embodiment of the invention, the population of cells may be obtained from the subject into whom the Tr1 cells-enriched composition is subsequently introduced.
In one embodiment of the invention, the subject can be one in which suppression of an immune response is desired.
In one embodiment, the subject is a human affected by an inflammatory condition or disease/disorder, such as any of the diseases/disorders including, but not limited to, non-autoimmune inflammatory bowel disease, post-surgical adhesions, coronary artery disease, hepatic fibrosis, acute respiratory distress syndrome, acute inflammatory pancreatitis, endoscopic retrograde cholangiopancreatography-induced pancreatitis, burns, atherogenesis of coronary, cerebral and peripheral arteries, appendicitis, cholecystitis, diverticulitis, visceral fibrotic disorders, wound healing, skin scarring disorders (keloids, hidradenitis suppurativa), granulomatous disorders (sarcoidosis, primary biliary cirrhosis), asthma, pyoderma gandrenosum, Sweet's syndrome, Behcet's disease, primary sclerosing cholangitis, and an abscess. In another embodiment, the subject is a human affected by an autoimmune condition or disease/disorder including, but not limited to, lupus erythematosus, pemphigus vulgaris, thyreoiditis, thrombocytopenic purpura, Graves disease, diabetes mellitus, juvenile diabetes, spontaneous autoimmune diabetes, myasthenia gravis, Addison's disease, an arthritic condition such as rheumatoid arthritis, ankylosing spondylitis, juvenile idiopathic arthritis, psoriatic arthritis; multiple sclerosis, psoriasis, uveitis, autoimmune hemolytic anemia, scleroderma, an intestinal inflammatory condition such as autoimmune inflammatory bowel disease, Crohn's disease, colitis, intestinal inflammation linked to food allergy; Sjogren's disease, Hashimoto's disease, myasthenia gravis, Autoimmune Polyendocrinopathy syndromes, Type I diabetes mellitus (TIDM), autoimmune gastritis, autoimmune uveoretinitis, polymyositis, and thyroiditis, as well as in the generalized autoimmune diseases typified by human Lupus. "Autoantigen" or "self-antigen" as used herein refers to an antigen or epitope which is native to the mammal and which is immunogenic in said mammal disease. The cells of the invention can also be used to prevent or treat transplantation reactions such as graft versus host disease (GVHD), hematopoietic stem cell transplantation and graft rejections.
In another embodiment, the subject is affected by an allergic or asthmatic condition. Examples of allergic or asthmatic condition include, but are not limited to, asthma, atopic dermatitis, allergic rhinitis, conjunctivitis, eczema, contact allergy, inhaled allergy, ingested allergy and anaphylaxis.

Introduction of Tr1 cells into patients is performed using methods well known in the art such as adoptive cell transfer. Briefly, a mixed population of cells is extracted from a target donor. Depending on the application, the cells may be extracted during a period of remission, or during active disease. Typically this is done by withdrawing whole blood and harvesting granulocytes by leukapheresis (leukopheresis). For example, large volume leukapheresis (LVL) has been shown to maximize blood leukocyte yield. The harvested lymphocytes may be separated using the cell separation techniques based on Tr1-specific cell markers such as those described herein, and then transfused to a patient, typically the cell donor (except in GVHD where the donor and recipient are different), for adoptive immune suppression. Approximately 10³ to 10¹¹, preferably 10⁴ to 10¹⁰, preferably 10⁵ to 10⁹, preferably 10⁶ to 10⁹, more preferably 10⁶ to 10⁸ Tr1 cells are injected into the patient.
As used herein, the term "effective amount" means the amount of a therapeutic substance or composition which is sufficient to reduce (to any extent) or ameliorate the severity and/or duration of a disorder or one or more symptoms thereof, prevent the advancement of a disorder, cause regression of a disorder, prevent the recurrence, development, onset or progression of one or more symptoms associated with a disorder, detect a disorder, or enhance or improve the prophylactic or therapeutic effect(s) of another therapy (e.g., prophylactic or therapeutic agent). The effective amount will vary with the age, gender, race, species, general condition, etc., of the subject, the severity of the condition being treated, the particular agent administered, the duration of the treatment, the nature of any concurrent treatment, the pharmaceutically acceptable carrier used, and like factors within the knowledge and expertise of those skilled in the art. As appropriate, an "effective amount" in any individual case can be determined by one of ordinary skill in the art by reference to the pertinent texts and literature and/or by using routine experimentation, (for example, see Gennaro et al., Eds. Remington's The Science and Practice of Pharmacy, 20th edition, (2000), Lippincott Williams and Wilkins, Baltimore MD; Braunwald et al., Eds. Harrison's Principles of Internal Medicine, 15th edition, (2001), McGraw Hill, NY; Berkow et al., Eds. The Merck Manual of Diagnosis and Therapy, (1992), Merck Research Laboratories, Rahway NJ).
The terms "prevent," "preventing," and "prevention" refer herein to the inhibition of the development or onset of a disorder or the prevention of the recurrence, onset, or development of one or more symptoms of a disorder in a subject resulting from the administration of a therapy (e.g., a prophylactic or therapeutic agent), or the administration of a combination of therapies (e.g., a combination of prophylactic or therapeutic agents). By "treatment" or "treating" is meant that at least an amelioration of the symptoms associated with the disorder in the host is achieved, where amelioration is used in a broad sense to refer to at least a reduction in the magnitude of a parameter, e.g. symptom, associated with the condition being treated. As such, treatment also includes situations where the pathological condition, or at least symptoms associated therewith, are completely inhibited, e.g. prevented from happening, or stopped, e.g. terminated, such that the host no longer suffers from the condition, or at least the symptoms that characterize the condition.
As used herein, the term "subject" refers to an animal, preferably a mammal and most preferably a human.

Another object of the invention is a method for increasing immune response of a transplanted cell population in a subject in need thereof, comprising administering to said subject an effective amount of a cell population depleted of Tr1 cells, resting Tr1 cells or activated Tr1 cells as described here above.
Another object of the invention is a method for increasing immune response of a transplanted cell population in a subject in need thereof, comprising administering to said subject an effective amount of a cell population depleted of resting Tr1 cells and/or activated Tr1 cells as described here above.
Accordingly, the absence of Tr1 cells in the transplanted cell population allows an increase in the desired immune response as no Tr1 cell performs its suppressive activity.

In one embodiment, said method is for treating a subject undergoing cancer treatment.
In one embodiment, the transplanted cell population is an antigen-specific effector T cell population. Preferably, said T cell population is specific for a tumor antigen such as MART-1 (Melan A) of melanoma or MAGE 1, 2, 3 of melanoma, thyroid medullary, small cell lung cancer, colon and/or bronchial squamous cell cancer...
In said embodiment, the cell population to be transplanted is depleted in resting and/or activated Tr1 cells in vitro before being administrated to the patient to be treated.

Depletion of regulatory T cells has been shown to enhance vaccine-mediated immunity in cancer patients (Dannull et al., 2005, 115(12):3623-3633). Thus, in another embodiment of the invention, it is proposed that depletion of Tr1 cells, resting Tr1 cells and/or activated Tr1 cells may be used in cell therapy for treating cancer or infectious disease wherein said Tr1 cells may be deleterious or harmful for the treatment. For example, in cancer treatment, a transient depletion of Tr1 cells could be interesting before a vaccination/immunotherapy protocol, said transient depletion being obtained by depleting the blood of a patient ex vivo through a device according to the depletion method of the invention and re-injecting it immediately to the patient. Thus, in said embodiment, the depletion of resting and/or activated Tr1 cells from the blood of the patient is carried out ex vivo through a device.

Another object of the invention is a method for monitoring in vitro the effect of a therapy in a subject, comprising identifying the resting and/or activated Tr1 cell population in a biological sample before and after therapy, wherein an increase in the resting and/or activated Tr1 cell population after therapy corresponds to a response to therapy.
It is well known in the art that Tr1 cells are impaired in subjects suffering from autoimmune or inflammatory diseases such as multiple sclerosis, asthma, phemphigus vulgaris, and rheumatoid arthritis. Therefore, a subject wherein an increase in the resting and/or activated Tr1 cell population can be observed after therapy is a subject responding to therapy.
According to the invention, the identification of the resting and/or activated Tr1 cell population is performed as described here above.
In one embodiment, biological samples are obtained from the subject before therapy and at different times after therapy, for example every week, or every month during therapy.

### BRIEF DESCRIPTION OF THE FIGURES

**Figure 1****:** Surface Expression of CD25 and CD127 and Intracellular expression of Foxp3 on human nTreg and Tr1 cells. Results are expressed as a mean % of expressing cells on 4 different Tr1 and nTreg cell populations stimulated through ligation of the CD3 and CD28 molecule (Act) or cultured at a resting state (Res).
**Figure 2****:** Surface Expression CD62L on human nTreg and Tr1 cells. Results are expressed as a mean % of expressing cells on 4 different Tr1 and nTreg cell populations stimulated through ligation of the CD3 and CD28 molecule (Act) or cultured at a resting state (Res).
**Figure 3****:** (A) Surface Expression of CD62L, CD25 and CD127 on human nTreg and Tr1 cells. Results are expressed as a mean fluorescence intensity (MFI) of Tr1 and nTreg cells populations stimulated through ligation of the CD3 and CD28 molecule (Act) or cultured at a resting state (Res). (B) shows representative histograms of flow cytometry staining of CD62L, CD25 and CD127 on human nTreg and Tr1 cells.
**Figure 4****:** Surface Expression of CD62L on mouse activated nTreg and Tr1 cells. Results are expressed as a mean % of expressing cells.
**Figure 5****:** (A) Surface Expression of CD62L on CD4⁺CD25⁻CD127^{lo} cells. (B) Results are expressed in percentage of CD62L expressing cells in the CD4⁺CD25⁻CD127^{lo} population.

### EXAMPLES

### EXPERIMENTAL PROCEDURES

### Tr1 cell isolation

Tr1 cells were isolated as described in Brun et al, (International Immunopharmacology, 2009). Briefly, peripheral blood cells were separated by Ficoll density centrifugation and cultured at 2x10⁶ cells per ml in the presence of food antigen in order to allow the proliferation of food antigen-specific Tr1 cells. After 13 days of culture, cells were cloned by limiting dilution method during 3 weeks. Growing clones were then expanded using CD3/CD28 stimulatory agents and cytokines (IL-2 and IL-4). The Tr1 cell identity of the clones was assessed by the cytokine production profile of the cells showing high IL-10 production, intermediate production of IFN-γ, and low IL-4 production (see Brun et al, International Immunopharmacology, 2009). For the production of mouse Tr1 cells, splenocytes were activated with anti-CD3 and anti-CD28 monoclonal antibodies during 7 days in the presence of IL-10, anti-IL-12 and anti-IL-4. The resulting cell population was cloned on irradiated syngeneic splenocytes and anti-CD3 antibody during 3 weeks. The growing clones were assessed for their cytokine secretion profile. Clones that show high IL-10 production, intermediate production of IFN-γ, and low IL-4 production were identified as Tr1 cells.

### CD4+ T lymphocytes and Treg cell isolation

PBMC were isolated from buffy coat preparations derived from the whole blood of healthy volunteers, by density sedimentation on Ficoll-Plaque PLUS gradients (GE healthcare). Cells recovered from the gradient interface were washed twice in RPMI 1640 medium, counted, and immediately used for MACS and staining. Briefly, CD4+ T cells were negatively selected from the total PBMC using the CD4 isolation kit (Dynal), yielding a population of CD4+ cells with purity of 92-98%. The in-vitro expansion of purified CD4+ Treg cells was performed as described by Battaglia et al (The journal of Immunology, 2006). In brief, isolated CD4+ T cells are activated with anti-CD3/anti-CD28 Ab-coated magnetic beads (Dynabeads CD3 / CD28 T Cell expander, Dynal Invitrogen). Cells were cultured in the presence of X-vivo 15 medium supplemented with 5% pooled AB human serum (Sigma), 1% penicillin/streptomycin, in the presence of 100nM Rapamycin. Three rounds of stimulation of 7 days each were performed and IL-2 (100U/ml) was added starting from the 2nd round of stimulation.

### Flow cytometry studies

For flow cytometry studies on human cells, the following antibodies were used: PE-conjugated anti-CD127 (clone R34.34 from Beckman Coulter), FITC-labeled anti-CD4 Ab from Becton Dickinson (clone #RP4-T4), PeCy5-labeled anti-CD62L (clone Dreg56 from Becton Dickinson) and allophycocyanin-conjugated anti-CD25 (clone M-A251 from Becton Dickinson). Intracytoplasmic staining for human Foxp3 was performed using the PE-conjugated anti-Foxp3 Ab (clone 259 D/C7 from BD) and the intracytoplasmic staining kit (BD), according to the manufacturer's instructions. For Flow cytometry studies on mouse cells, the antibody used were PECy7- conjugated anti-CD4 (clone RM4-15), APC- conjugated anti-CD25 (clone PC61), PE- conjugated FoxP3 (clone MF23) and PE-conjugated anti-CD62L (clone Mel14). All anti-mouse antibodies were purchased from Becton Dickinson.

### Gene expression studies

Gene expression studies were performed using DNA microarray analysis as described in Dayem et al (Comp Funct Genom, 2003). For this purpose, Cell samples were lysed and the RNA stabilized with the Trizol Reagent (Invitrogen). The RNAs were purified with the RNeasy minikit from Qiagen and the residual amounts of DNA remaining were removed using a DNAse from Ambion (DNA-free). The concentration and the quality of the isolated RNA were determined with a NanoDrop (Thermo) and an Agilent Bioanalyzer 2100. The RNA were hybridized on human Affymetrix microarrays (Affymetrix-HuGene-1_0-st-v1).

### RESULTS

Natural CD4⁺ regulatory cells (nTreg cells) were first described by the constitutive expression of the CD25 molecule. Moreover, CD25 is also an activation marker for all types of T-cells and thus it was impossible to discriminate between nTreg cells and activated conventional T cells (ConvTcells). The same rules are also true for the expression of the master regulatory gene Foxp3 which expression is constitutive on nTreg cells and upregulated on activated convT cells.

Several years after the discovery of Foxp3 and CD25 as constitutive markers of Treg cells, the molecule CD127 has been shown to be constitutively low on the surface of Treg cells but constitutively highly expressed on the surface of all conventional T cells giving a method to discriminate these two populations also when activated.
∘ CD3⁺CD4⁺Foxp3⁺CD25⁺CD127^{lo} = nTreg cells (resting and activated)
∘ CD3⁺CD4⁺Foxp3⁺CD25⁺CD127^{hi} = Activated conv T cells
∘ CD3⁺CD4⁺Foxp3⁻CD25⁻CD127^{hi} = Resting conv Tcells

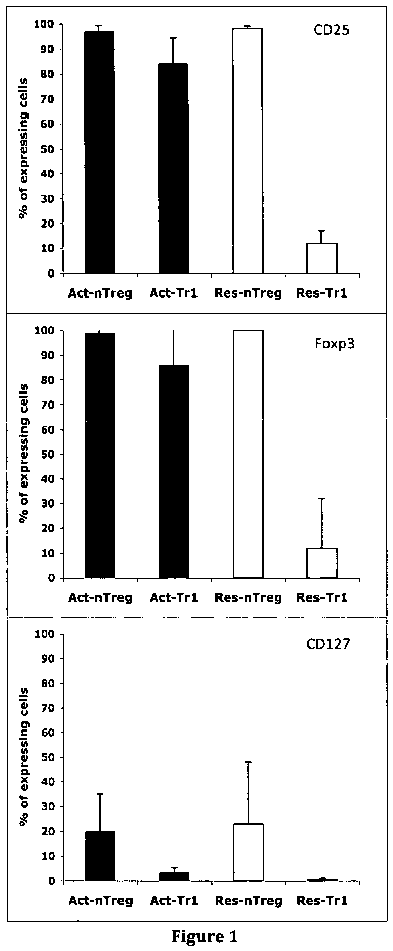

Beside nTreg cells, another well-studied regulatory cell population is also implicated in immune tolerance. This population called Tr1 lymphocytes was also phenotyped by the expression of the above molecule. The results are the following:
∘ CD3⁺CD4⁺Foxp3⁺CD25⁺CD127^{lo} = Activated Tr1 cells
∘ CD3⁺CD4⁺Foxp3⁻CD25⁻CD127^{lo} = Resting Tr1 cells
This results show that resting Tr1 cells can be discriminated from nTreg cells by the expression of CD25 and CD127, or by the expression of Foxp3, CD25 and CD127. However Activated Tr1 cells demonstrate the same phenotype as nTreg cells (Figure 1).

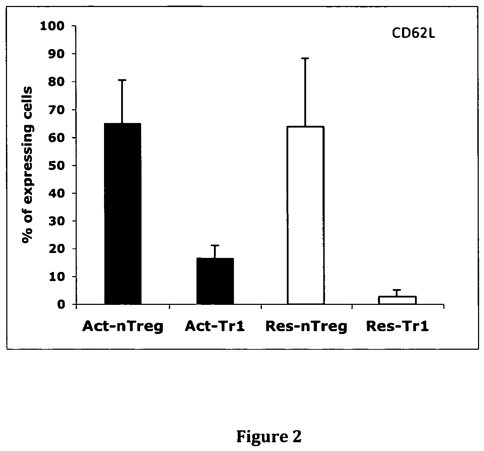

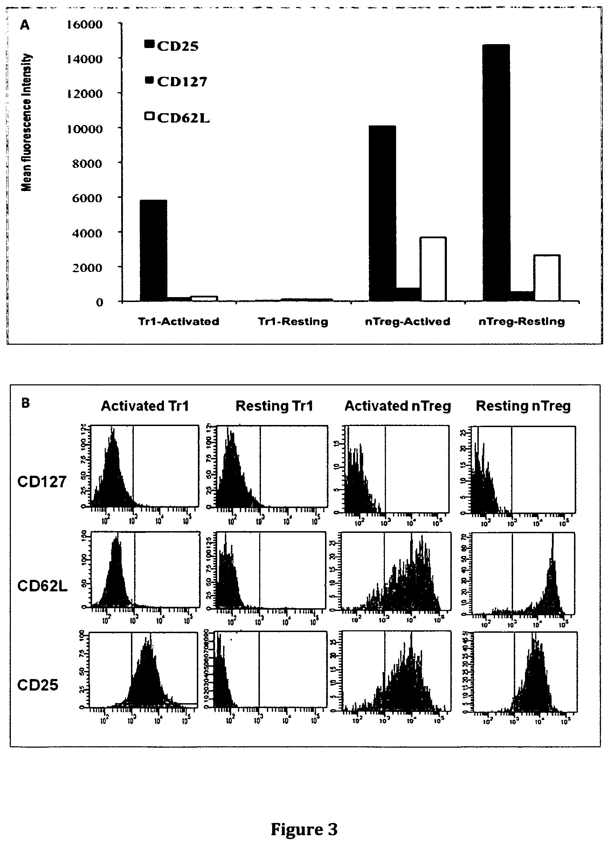

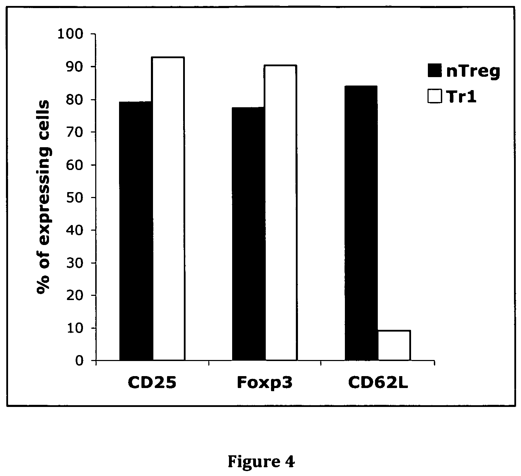

In order to test whether another surface molecule can allow the discrimination of these two populations, we tested the number of cells expressing CD62L on the surface of both human nTreg and Tr1 cells. A high number of both resting and activated Treg cells express CD62L, whereas a low number of Tr1 cells either resting or activated express CD62L (Figure 2). Importantly the mean fluorescence intensity (MFI) of CD62L, CD127 and CD25 staining show that, as previously reported, nTreg are constitutively expressing CD62L. In contrast, CD62L is constitutively absent from Tr1 cells (Figure 3). Thus, CD62L phenotyping can discriminate between activated Tr1 cells and nTreg cells when included in the above panel of markers. The MFI of CD127 staining is low for all four cell populations. The MFI of CD25 staining is constitutively high on nTreg cells, is high on activated Tr1 cells and downregulated to low MFI on resting Tr1 cells. This observation was also confirmed by RT-PCR experiments (Figure 4).
∘ CD3⁺CD4⁺Foxp3⁻CD25⁻CD127^{lo}CD62L^{lo} = resting Tr1 cells
∘ CD3⁺CD4⁺Foxp3⁺CD25⁺CD127^{lo}CD62L^{lo} = activated Tr1 cells
∘ CD3⁺CD4⁺Foxp3⁺CD25⁺CD127^{lo}CD62L^{hi} = nTreg cells
Figure 4 shows that the discriminating role of surface CD62L between activated Tr1 cells and nTreg cells is also true for cell population originated from mice.

Peripheral blood leucocytes from two healthy human individuals were stained with fluorescent labeled CD4, CD25, CD127 and CD62L specific antibodies. The percentage of cells expressing CD62L was determined on the subset of cells characterized by a CD4⁺CD25⁻CD127^{lo} phenotype.

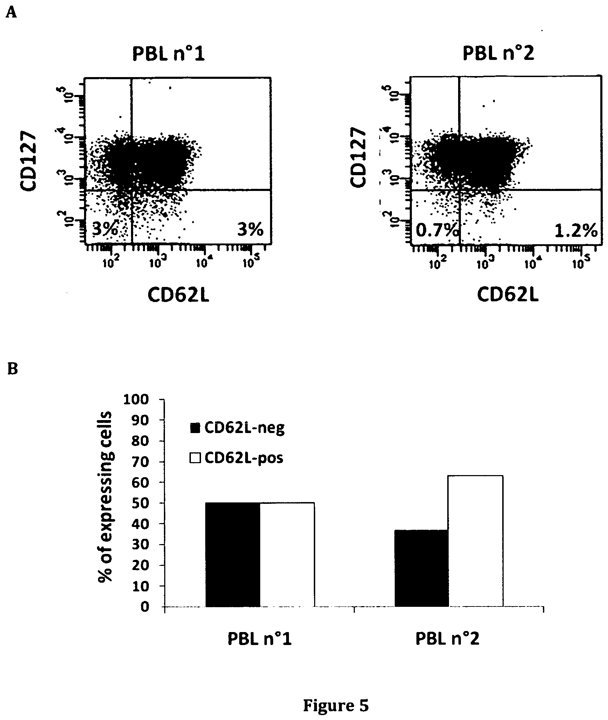

Figure 5A and B shows that in the CD4⁺CD25⁻CD127^{lo} population, the marker CD62L allows to discriminate a CD62L^{lo} population and a CD62L^{hi} population. Therefore, the markers CD4, CD25 and CD127 are not sufficient for discriminating resting Tr1 cells in a cell population.

In conclusion, Table 1 shows how to discriminate each population depending on the markers used.

**Table 1**

| | Resting conv. T cells | Activated conv. T cells | Resting and activated nTreg | Resting Tr1 cells | Activated Tr1 cells |
|---|---|---|---|---|---|
| CD4 | + | + | + | + | + |
| CD25 | - | + | + | - | + |
| CD127 | + | + | low | low | low |
| CD62L | + | - | high | low | low |

Foxp 3 may also be used optionally for discriminating cell populations:

**Table 2**

| | Resting conv. T cells | Activated conv. T cells | Resting and activated nTreg | Resting Tr1 cells | Activated Tr1 cells |
|---|---|---|---|---|---|
| FOXP3 | - | + | + | - | + |

## Claims

1. A method of identifying a resting Tr1 cell population, comprising detecting the cell surface expression of CD4, CD25, CD127 and CD62L markers on a cell population, wherein the cells expressing:
- CD4, and
- a low level of CD127,
- a low level of CD62L.
and not expressing:
- CD25,
are the resting Tr1 cells.

2. A method of identifying an activated Tr1 cell population, comprising detecting the cell surface expression of CD4, CD25, CD127 and CD62L markers on a cell population, wherein the cells expressing:
- CD4,
- CD25,
- a low level of CD127, and
- a low level of CD62L,
are the activated Tr1 cells.

3. A method for isolating a resting or an activated Tr1 cell population, comprising
- identifying a resting Tr1 cell population or an activated Tr1 cell population according to claim **1** or **2**, and
- isolating said population.

4. An isolated population of resting Tr1 cells having the phenotype CD4⁺CD25⁻CD127^{low}CD62L^{low}.

5. An isolated population of activated Tr1 cells having the phenotype CD4⁺CD25⁺CD127^{low}CD62L^{low}.

6. A method for enriching a cell population in resting and/or activated Tr1 cells, comprising:
- identifying the resting and/or activated Tr1 cells according to claim **1** or **2,**
- selecting said cells,
thereby obtaining a cell population enriched in resting and/or activated Tr1 cells wherein the percentage of resting and/or activated Tr1 cells is at least twice the percentage of resting and/or activated Tr1 cells before enrichment.

7. An enriched population of resting Tr1 cells having the phenotype CD4⁺CD25⁻CD127^{low}CD62L^{low} or activated Tr1 cells having the phenotype CD4⁺CD25⁺CD127^{low}CD62L^{low}, wherein at least 50% of the cells of the enriched population of resting or activated Tr1 cells are Tr1 cells.

8. An enriched population of resting Tr1 cells having the phenotype CD4⁺CD25⁻CD127^{low}CD62L^{low} and activated Tr1 cells having the phenotype CD4⁺CD25⁺CD127^{low}CD62L^{low}, wherein at least 50% of the cells of the enriched population of resting and activated Tr1 cells are Tr1 cells.

9. A method for depleting a cell population in resting Tr1 cells and/or activated Tr1 cells, comprising:
- identifying the resting Tr1 cells according to claim **1** and/or the activated Tr1 cells according to claim **2**,
- depleting said cells,
thereby obtaining a cell population depleted in resting and/or activated Tr1 cells, wherein the percentage of resting and/or activated Tr1 is at most 0.75 fold the percentage of resting and/or activated Tr1 cells before depletion.

10. A pharmaceutical composition comprising an isolated resting Tr1 cell population according to claim **4** or an isolated activated Tr1 cell population according to claim **5** or an enriched resting or activated Tr1 cell population according to claim 7.

11. The pharmaceutical composition according to claim **10** for use in preventing or treating an immune response associated with an infection or transplantation or an allergic disease or for use in preventing or treating an inflammatory condition or an autoimmune condition.

12. A pharmaceutical composition comprising a mixture of resting Tr1 cells and activated Tr1 cells, wherein the resting Tr1 cells have the phenotype CD4⁺CD25⁻CD127^{low}CD62L^{low} and the activated Tr1 cells have the phenotype CD4⁺CD25⁺CD127^{low}CD62L^{low}.

13. The pharmaceutical composition according to claim **12** for use in treating an immune response associated with an infection or transplantation or an allergic disease or for use in preventing or treating an inflammatory condition or an autoimmune condition.

14. A method for monitoring in vitro the effect of a therapy against an autoimmune or inflammatory disease in a subject, comprising identifying the resting and/or activated Tr1 cell population in a biological sample obtained from the subject before and after said therapy according to the method of claim **1** or claim **2**, wherein an increase in the resting and/or activated Tr1 cell population after said therapy corresponds to a response to said therapy.

## Patentansprüche

1. Verfahren zum Identifizieren einer ruhenden Tr1-Zellpopulation, umfassend Nachweisen einer Zelloberflächenexpression von CD4-, CD25-, CD127- und CD62L-Markern auf einer Zellpopulation, wobei die Zellen, die Folgendes exprimieren:
- CD4, und
- ein niedriges Niveau von CD127,
- ein niedriges Niveau von CD62L,
und nicht Folgendes exprimieren:
- CD25,
ruhende Tr1-Zellen sind.

2. Verfahren zum Identifizieren einer aktivierten Tr1-Zellpopulation, umfassend Nachweisen der Zelloberflächenexpression von CD4-, CD25-, CD127- und CD62L-Markern auf einer Zellpopulation, wobei die Zellen, die Folgendes exprimieren:
- CD4,
- CD25,
- ein niedriges Niveau von CD127, und
- ein niedriges Niveau von CD62L,
aktivierte Tr1-Zellen sind.

3. Verfahren zum Isolieren einer ruhenden oder einer aktivierten Tr1-Zellpopulation, umfassend:
- Identifizieren einer ruhenden Tr1-Zellpopulation oder einer aktivierten Tr1-Zellpopulation nach Anspruch **1** oder **2**, und
- Isolieren der Population.

4. Isolierte Population von ruhenden Tr1-Zellen mit dem Phänotyp CD4⁺CD25⁻ CD127^{low}CD62L^{low}.

5. Isolierte Population von aktivierten Tr1-Zellen mit dem Phänotyp CD4⁺CD25⁺CD127^{low}CD62L^{low}.

6. Verfahren zum Anreichern einer Zellpopulation in ruhenden und/oder aktivierten Tr1-Zellen, umfassend:
- Identifizieren der ruhenden und/oder aktivierten Tr1-Zellen nach Anspruch **1** oder **2**,
- Auswählen der Zellen,
dadurch Erhalten einer Zellpopulation, die in ruhenden und/oder aktivierten Tr1-Zellen angereichert ist, wobei der Prozentsatz von ruhenden und/oder aktivierten Tr1-Zellen mindestens zweimal der Prozentsatz von ruhenden und/oder aktivierten Tr1-Zellen vor der Anreicherung ist.

7. Angereicherte Population von ruhenden Tr1-Zellen mit dem Phänotyp CD4⁺CD25⁻ CD127^{low}CD62L^{low} oder aktivierte Tr1-Zellen mit dem Phänotyp CD4⁺CD25⁺CD127^{low}CD62L^{low}, wobei mindestens 50% der Zellen der angereicherten Population von ruhenden oder aktivierten Tr1-Zellen Tr1-Zellen sind.

8. Angereicherte Population von ruhenden Tr1-Zellen mit dem Phänotyp CD4⁺CD25⁻ CD127^{low}CD62L^{low} und aktivierte Tr1-Zellen mit dem Phänotyp CD4⁺CD25⁺CD127^{low}CD62L^{low}, wobei mindestens 50% der Zellen der angereicherten Population von ruhenden oder aktivierten Tr1-Zellen Tr1-Zellen sind.

9. Verfahren zum Depletieren einer Zellpopulation in ruhenden Tr1-Zellen und/oder aktivierten Tr1-Zellen, umfassend:
- Identifizieren der ruhenden Tr1-Zellen nach Anspruch **1**, und/oder der aktivierten Tr1-Zellen nach Anspruch **2**,
- Depletieren der Zellen,
dadurch Erhalten einer Zellpopulation, die in ruhenden und/oder aktivierten Tr1-Zellen depletiert ist, wobei der Prozentsatz von ruhenden und/oder aktivierten Tr1-Zellen höchstens 0,75 Mal der Prozentsatz von ruhenden und/oder aktivierten Tr1-Zellen vor der Depletion ist.

10. Pharmazeutische Zusammensetzung, umfassend eine isolierte ruhende Tr1-Zellpopulation nach Anspruch **4** oder eine isolierte aktivierte Tr1-Zellpopulation nach Anspruch **5** oder eine angereicherte ruhende oder aktivierte Tr1-Zellpopulation nach Anspruch **7**.

11. Pharmazeutische Zusammensetzung nach Anspruch **10**, zur Verwendung bei der Prävention oder Behandlung einer Immunantwort, die mit einer Infektion oder Transplantation oder allergischen Erkrankung assoziiert ist, oder zur Verwendung bei der Prävention oder Behandlung eines entzündlichen Leidens oder eines autoimmunen Leidens.

12. Pharmazeutische Zusammensetzung, umfassend eine Mischung aus ruhenden Tr1-Zellen oder aktivierten Tr1-Zellen, wobei die ruhenden Tr1-Zellen den Phänotyp CD4⁺CD25⁻CD127^{low}CD62L^{low} und die aktivierten Tr1-Zellen den Phänotyp CD4⁺CD25⁺CD127^{low}CD62L^{low} haben.

13. Pharmazeutische Zusammensetzung nach Anspruch **12**, zur Verwendung bei der Behandlung einer Immunantwort, die mit einer Infektion oder Transplantation oder allergischen Erkrankung assoziiert ist, oder zur Verwendung bei der Prävention oder Behandlung eines entzündlichen Leidens oder eines autoimmunen Leidens.

14. Verfahren zur Überwachung eines in vitro-Effekts einer Therapie gegen eine autoimmune oder entzündliche Erkrankung in einem Subjekt, umfassend das Identifizieren der ruhenden und/oder aktivierten Tr1-Zellpopulation in einer biologischen Probe, erhalten vom Subjekt vor und nach der Therapie nach dem Verfahren von Anspruch **1** oder Anspruch **2**, wobei eine Erhöhung der ruhenden und/oder aktivierten Tr1-Population nach der Therapie einer Antwort auf die Therapie entspricht.

## Revendications

1. Un procédé d'identification d'une population cellulaire Tr1 au repos, comprenant la détection de l'expression à la surface cellulaire des marqueurs CD4, CD25, CD127 et CD62L sur une population cellulaire, dans lequel les cellules exprimant :
- CD4, et
- un faible niveau de CD127,
- un faible niveau de CD62L,
et n'exprimant pas :
- CD25,
sont les cellules Tr1 au repos.

2. Un procédé d'identification d'une population cellulaire Tr1 activée, comprenant la détection de l'expression à la surface cellulaire des marqueurs CD4, CD25, CD127 et CD62L sur une population cellulaire, dans lequel les cellules exprimant :
- CD4,
- CD25,
- un faible niveau de CD127, et
- un faible niveau de CD62L,
sont les cellules Tr1 activées.

3. Un procédé pour isoler une population cellulaire Tr1 au repos ou activée, comprenant :
- l'identification d'une population cellulaire Tr1 au repos ou d'une population cellulaire Tr1 activée selon la revendication **1** ou **2**, et
- l'isolation de ladite population.

4. Une population isolée de cellules Tr1 au repos ayant le phénotype CD4⁺CD25⁻ CD127^{low}CD62L^{low}.

5. Une population isolée de cellules Tr1 activées ayant le phénotype CD4⁺CD25⁺CD127^{low}CD62L^{low}.

6. Un procédé pour enrichir une population cellulaire en cellules Tr1 au repos et/ou activées, comprenant :
- l'identification de cellules Tr1 au repos et/ou activées selon la revendication **1** ou **2**,
- la sélection desdites cellules,
obtenant ainsi une population cellulaire enrichie en cellules Tr1 au repos et/ou activées dans laquelle le pourcentage de cellules Tr1 au repos et/ou activées est au moins deux fois le pourcentage de cellules Tr1 au repos et/ou activées avant enrichissement.

7. Une population enrichie en cellules Tr1 au repos ayant le phénotype CD4⁺CD25⁻ CD127^{low}CD62L^{low} ou en cellules Tr1 activées ayant le phénotype CD4⁺CD25⁺CD127^{low}CD62L^{low}, dans laquelle au moins 50% des cellules de la population enrichie en cellules Tr1 au repos ou activées sont des cellules Tr1.

8. Une population enrichie en cellules Tr1 au repos ayant le phénotype CD4⁺CD25⁻ CD127^{low}CD62L^{low} et en cellules Tr1 activées ayant le phénotype CD4⁺CD25⁺CD127^{low}CD62L^{low}, dans laquelle au moins 50% des cellules de la population enrichie en cellules Tr1 au repos et activées sont des cellules Tr1.

9. Un procédé pour dépléter une population cellulaire en cellules Tr1 au repos et/ou activées, comprenant :
- l'identification de cellules Tr1 au repos selon la revendication **1** et/ou de cellules Tr1 activées selon la revendication **2**,
- la déplétion desdites cellules,
obtenant ainsi une population cellulaire déplétée en cellules Tr1 au repos et/ou activées, dans laquelle le pourcentage de cellules Tr1 au repos et/ou activées est au plus 0.75 fois le pourcentage de cellules Tr1 au repos et/ou activées avant déplétion.

10. Une composition pharmaceutique comprenant une population isolée de cellules Tr1 au repos selon la revendication **4** ou une population isolée de cellules Tr1 activées selon la revendication **5** ou une population enrichie en cellules Tr1 au repos ou activées selon la revendication **7.**

11. La composition pharmaceutique selon la revendication **10** pour son utilisation dans la prévention ou le traitement d'une réponse immunitaire associée à une infection ou à une transplantation ou à une maladie allergique ou pour son utilisation dans la prévention ou le traitement d'un état inflammatoire ou d'un état auto-immun.

12. Une composition pharmaceutique comprenant un mélange de cellules Tr1 au repos et de cellules Tr1 activées, dans laquelle les cellules Tr1 au repos ont le phénotype CD4⁺CD25⁻CD127^{low}CD62L^{low} et les cellules Tr1 activées ont le phénotype CD4⁺CD25⁺CD127^{low}CD62L^{low}.

13. La composition pharmaceutique selon la revendication **12** pour son utilisation dans le traitement d'une réponse immunitaire associée à une infection ou à une transplantation ou à une maladie allergique ou pour son utilisation dans la prévention ou le traitement d'un état inflammatoire ou d'un état auto-immun.

14. Un procédé pour suivre l'effet in vitro d'une thérapie contre une maladie autoimmune ou inflammatoire chez un sujet, comprenant l'identification d'une population cellulaire Tr1 au repos et/ou activée dans un échantillon biologique obtenu du sujet avant et après ladite thérapie selon le procédé de la revendication **1** ou de la revendication **2**, dans lequel une augmentation de la population cellulaire Tr1 au repos et/ou activée après ladite thérapie correspond à une réponse à ladite thérapie.
